# EUROPEAN PATENT SPECIFICATION

(11) **EP 2 004 795 B1**
(45) Date of publication and mention of the grant of the patent: **01.09.2010**
(21) Application number: 07775039.6
(22) Date of filing: 11.04.2007
(51) Int. Cl.: C12M 1/00, C12M 1/34

(54) **INCUBATION CONDITION MONITORING DEVICE**
VORRICHTUNG ZUR ÜBERWACHUNG VON INKUBATIONSBEDINGUNGEN
DISPOSITIF DE SURVEILLANCE DE CONDITIONS D'INCUBATION

(30) Priority: 11.04.2006 US 791159 P
(43) Date of publication of application: 24.12.2008
(73) Proprietor: William A. Cook Australia Pty. Ltd., Brisbane, Queensland 4113 (AU); Cook Urological Inc., Spencer IN 47460 (US)
(72) Inventor: SPITTLE, Jason, William, Balmoral, Queensland, 4171 (AU); DAVIS, Steve, Charles, Ascot, Queensland, 4007 (AU); HINSCH, Andrew, Mansfield, Queensland, 4122 (AU); HUBERTS, John, Sunnybank Hills, Queensland, 4109 (AU)
(74) Representative: Garratt, Peter Douglas
(86) International application number: PCT/US2007/008777
(87) International publication number: WO 2007/120619

(56) References cited:
- WO-A-03/085101
- WO-A-2007/001248
- US-A1- 2003 092 178
- US-A1- 2004 212 285
- US-A1- 2005 244 306

## Description

### Technical Field

This invention relates to cell culturing and more particularly to monitoring of conditions during cell culturing. Background of the Invention

In cell culturing applications such as in vitro fertilisation cell culture, it is important to regulate the environment to ensure healthy cell growth. Two of the important parameters are temperature and pH of the culture media. The invention will be discussed in relation to monitoring of these two parameters but the invention is not so limited and can be applied to the monitoring of other relevant parameters.

Temperature is maintained and regulated during the culture cycle with the aid of an incubator. However, at various stages the culture vessels are removed from the incubator to make observations, change media etc., and during this time there is typically no monitoring of the temperature changes to which the vessels are being subjected. If the temperature goes outside of a particular range there can be deleterious consequences for the cells. Similarly in the case of pH there is little monitoring of changes, other than in some cases the use of visual indicators such as phenol red added to the media. However, the optimal pH range required is quite narrow and changes in colour can be difficult to pick up with the naked eye.

pH in incubators is often regulated with carbon dioxide gas, but currently there is no feedback system which monitors the pH of the media.

Reference is directed to WO-A-03085101 which discloses an incubator comprising sensors to measure selected characteristics within the incubator. The data collected by the sensor are transferred to a computer for storage and processing. Reference is also directed to US-A-2003/0092178, US-A-2005/0244306 and US-A-2004/0212285, which describe incubators provided with monitoring systems comprising a sensor for essential parameters, transition mechanisms for the collected data and monitoring, displaying and storing devices for said data.

### Summary of the Invention

The present invention consists in a temperature and pH logging system for cell culture to provide an audit history and/or control feedback for incubation conditions comprising at least one reader unit for measuring pH and temperature associated with the culture media in a culture vessel in an incubator, a data logger for recording and storing the measured pH and temperature to provide the audit history and a warning system if temperature or pH goes outside a specified range, wherein the reader unit comprises a cuvette containing the same culture media as the culture vessel and the measurement of the selected characteristic is carried out in the cuvette.

In one embodiment the incubator may contain a plurality of culture vessels and each culture vessel may include a reader unit.

Each reader unit can include wireless transmission means to transmit the measurements to a data logger when the culture vessel is inside an incubator and also when the vessel is outside the incubator. A state of being outside the incubator may be determined by a photo detector registering light or a change in ambient light.

Preferably each reader unit comprises a gripper arrangement to grip and retain the culture vessel and a separate cuvette containing the same medium as the culture vessel and the measurement of the selected characteristics is carried out in the cuvette. As both the culture vessel and the cuvette are in the same environment, the measurement of conditions in the cuvette equate to measurement of conditions within the culture vessel without the need for any probes to be placed into directly into the culture vessel.

Preferably each culture vessel includes a thermocouple to read temperature.

Preferably the monitor and display system includes and alarm system to provide an alarm when the measured characteristics are outside a selected range and wherein the alarm can be selected from audible, visual or any other suitable means or combination thereof.

The reader can include optical devices to measure pH by reading the colour of a pH indicator, and the indicator may be in solution or immobilised on a suitable surface. In one embodiment the reader utilises measurements at three wavelengths, two of which show appreciable absorption by the acid and base forms of the indicator, and a third wavelength which shows little absorption by either form of the indicator and can be used to correct for offsets in the zero level. An additional zero point correction can be applied to account for the effect of temperature using the temperature measurement.

The receiver/transmitter within the incubator can transmit the measurements of the selected characteristics to the data logger wirelessly or by hard wiring.

Preferably the pH is measured using an optical method of pH measurement comprising; using a coloured pH indicator(s) such as phenol red (the indicators may be in solution or immobilised on a surface); using three or more wavelengths where two or more are used to derive the pH from a ratio of the acid and base forms of the indicator, so is substantially independent of the amount of indicator present; one or more wavelengths having little absorption by either form of the indicator are used to track and correct for offset changes in zero point conditions; determining calibration coefficients for the zero point as a function of temperature for each of the wavelengths, and applying a zero point correction in conjunction with the temperature measurement, whereby the combination of temperature correction and offset correction of the zero point means it is not necessary to take a zero reading of the solution prior to each measurement.

Preferably each measurement unit is attached to (or situated within) a particular culture vessel to monitor changes associated with that vessel wherever it is situated.

Preferably the measurement unit transmits data wirelessly. Hence it will be seen that the concept is to produce a small unit which can be used to monitor pH and temperature conditions inside an incubator, and can also travel with a culture vessel outside the incubator. The monitoring inside the incubator can also be used as feedback for a control system.

Ideally the unit would be small enough to fit inside the culture vessel itself, however, a unit small enough to fit inside micro droplets (or even for example a well of a Nunc four well dish) is not practical at this time using off the shelf components. The unit envisaged is expected to be more like 30 mm square, but the principle described could in future be implemented on a micro scale when technology allows.

The next best option is to have a unit which monitors a cuvette containing the same solutions as in the culture dish, but is external to the dish. The pH can be measured optically and an optical method has the advantage of not suffering significant drift. Conventional pH probes need frequent re-calibration and will drift, particularly in solutions containing compounds like proteins. Phenol red indicator is one pH indicator which can be used to monitor the required pH range.

Hence in a further form of the invention the reader unit comprises a reader body, a recess in the reader body to receive a cuvette, a LED to transmit light through the recess, a LED receiver to receive light transmitted through the recess, a thermocouple to measure temperature, an electronic circuit to receive and transmit signals from the LED receiver and the thermocouple.

Preferably the reader unit further includes a gripper to grip and retain a culture vessel to the reader unit.

Preferably the LED arrangement to transmit light comprises at least 3 LEDs providing three or more wavelengths where at least two are used to derive the pH from a ratio of the acid and base forms of a colour indicator and one wavelengths is used to track and correct for offset changes in zero point conditions and preferably the LED receiver arrangement to receive light transmitted through the recess comprises receivers for each of the frequencies of the LED arrangement.

### Brief Description of the Drawing

This then generally describes the invention but to assist with understanding reference will now be made to the accompanying drawings in which:
Figure 1 shows a schematic view of one embodiment of the invention;
Figure 2 shows a perspective view of one embodiment of a reader unit of the present invention; and
Figure 3 shows a cutaway view of the reader unit of Figure 2.

### Detailed Description

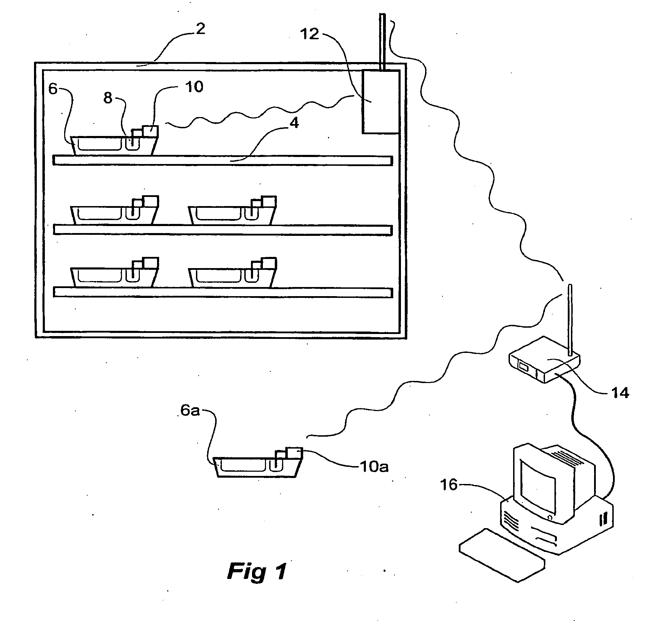

A preferred embodiment of the invention as shown in Figure 1 comprises an incubator 2 which has trays 4 upon which are carried culture dishes 6. Other culture vessels such as flasks may also be used. Similarly the incubator may be of any size or construction. Each culture dish is accompanied by a pH and a temperature sensors associated with a cuvette 8 of media. The sensors perform measurements of pH and temperature of the media in the cuvette and hence of the pH and temperature of the media in the culture dish without the need for the light sensors and thermocouple to be directed into the culture dish. Hereinafter these units are referred to as "reader units". In this preferred embodiment the reader units perform the pH measurement optically using light emitting diodes (LEDs) as the light source and temperature measurement using a thermocouple.

A preferred embodiment of the reader unit comprises a fully sealed unit so it can withstand spillages, with packaging made from a suitable plastic which can be cleaned and sterilised. In other cell culture applications using large dishes or flasks it would be possible to immerse the unit in the actual solution being monitored. In this case if the phenol red is not in the solution an optode with immobilised indicator would be used. The reader unit may be either re-chargeable, or have a battery which either lasts a sufficiently long time, or is replaceable.

The reader unit 10 has wireless communication capability to a slave receiver/transmitter unit 12. The slave receiver/transmitter unit 12 is connected wirelessly to a data logger 14 which records the data from the reader units. The data logger 14 has a download capability to a computer system 16 which displays and stores the details of temperature and pH. Alternatively the slave receiver/transmitter unit 12 can be hard wired to the data logger 14.

The complete system is modular and expandable. A central data logger is the repository of data and can accommodate the data streams from multiple readers. The data is downloadable to a PC and a suitable piece of software for downloading and presenting the data forms part of the system. Reader units can be used to monitor the conditions and control feedback in an incubator, but the use of a reader per culture vessel enables tracking of the history of the individual culture vessels. When the vessel is outside the incubator for inspection, media changes etc., it is most susceptible to variations in temperature and pH, so this is really the crucial time to be monitoring the situation. In such situations the reader unit 10a can transmit wirelessly directly to the data logger 14.

Since much of the culture cycle will be spent inside a metal clad incubator it is envisaged there can be a slave receiver/transmitter placed inside or outside the incubator to receive the wireless signals from the units during these periods. This unit can be connected to the main logger unit situated outside the incubator and may be connected wirelessly or be hard wired. Alternatively the reader unit may be hard wired to the data logger or the data logger may have antennae which are inserted into the incubator (thereby removing the need for slave receiver/transmitter units). However since the preferred embodiment is one where there is a central data logger receiving data from multiple incubators (and multiple dishes therein), greater flexibility would be provided by having a slave receiver/transmitter unit with each incubator. If the incubators were clad in material which transmits radio signals the receiver/transmitter could also directly transmit to the logger unit.

Hence, when the culture dish 6a is outside the incubator 2 then the reader unit 10a can transmit directly to the data logger 14.

In the case where the reader unit is being used to monitor the history of an individual vessel it needs to stay associated with that vessel, and a holder can be used which holds both the vessel and reader unit so they are transported about together.

In the preferred embodiment the reader units transmit data wirelessly. Whilst inside the incubator the data will be received by the slave unit. The main logger unit could also look for the data stream and will receive it when the units are outside the incubator, since the slave units will not receive the signals through the metal cladding of the incubator. Alternatively the slave receiver/transmitter unit may be placed on the outside of the incubator with an antenna inside and outside the incubator so it always receives the signal. To conserve power the reader unit whilst in the incubator may not transmit data continuously, but at a pre-determined time interval. Once the reader unit is outside the incubator the reader can transmit more frequently since this is the time when changes are likely to occur more rapidly. One way of having the reader know it is outside the incubator is to use a photodiode and look for changes in ambient light. Inside the incubator it will generally be dark. The reader units will also have warning indicators of when pH or temperature start to go outside of the acceptable range to warn the vessel should be put back in the incubator. If the cycle is complete and/or the dish is left out for a long period of time, the unit may revert back to a slower period of sampling.

### pH Measurement Principle

In the preferred embodiment the reader will use three wavelengths in the optical measurement (more than three could also be used). Two of these wavelengths will be used to determine the pH from the ratio of acid and base form concentrations of the indicator. This is determined using the absorption coefficients of the acid and base forms of the indicator and solving the simultaneous equations for the absorption at the two wavelengths. Using a ratio makes the measurement relatively independent of the actual amount of indicator added to the cuvette. Since the device is to be as low cost as possible it is another aspect of the invention to incorporate a method of auto zeroing. Normally in optical measurements a zero level measurement is performed with a sample blank prior to measuring the sample. The absorption levels of the blank are then subtracted from the sample reading to provide the net absorbance of the sample. In this device the third wavelength is chosen such that it shows very little absorption by the indicator and is used as a means of tracking changes in the zero level. Changes in the absorption level of this wavelength channel are then indicative of changes in the zero level, and the other two wavelengths being used in the measurement can be zero corrected on the basis of the changes measured at this third wavelength. This will correct for variations arising due to offsets, for example arising from different wall thickness cuvettes or coatings depositing out of solution onto the cuvette walls.

Another factor which affects the zero level is the temperature of the LEDs. Experiments have shown the intensities of the three wavelengths vary with temperature, but not by the same absolute amount. A simple factory calibration of the device provides coefficients for the relationship between the different wavelength LEDs. Any shift in the absorbance level of the third wavelength will be due to effects of offsets (as described above) and temperature drift. The measured temperature can be used to calculate the thermal drift component, and the remainder of any change in the zero level of the third wavelength will be due to offset effects. The offset and temperature drift corrections can then be determined and applied to the other two wavelengths used in determining the pH.

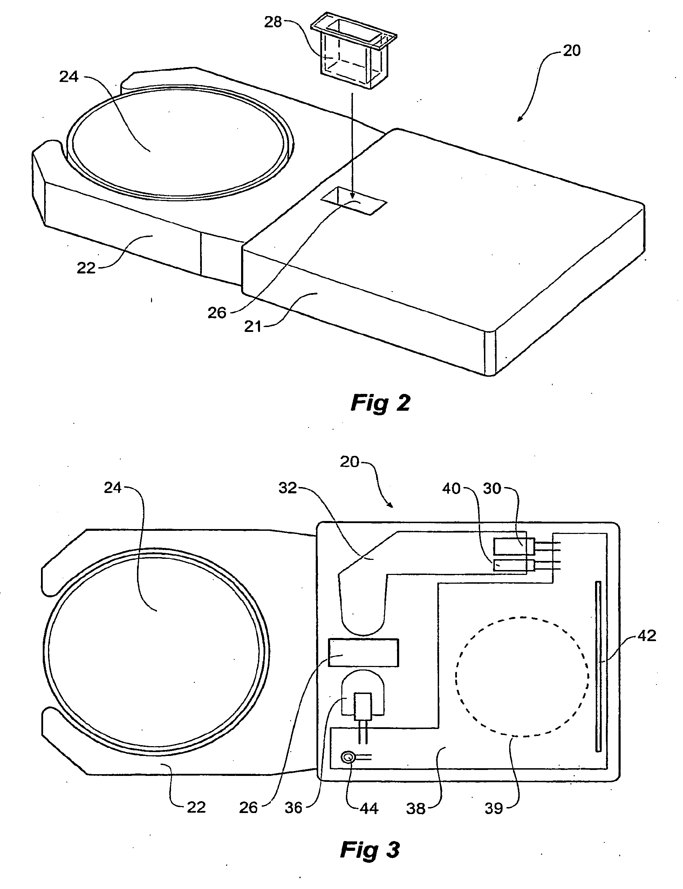

Figure 2 shows one embodiment the of reader unit according to the present invention. The reader unit 20 has a reader body 21 and a gripper 22 to receive and retain a culture vessel 24. The gripper may be of any convenient size to grip and carry a culture vessel. For instance the gripper may be made of silicone elastomer and be sized to grip and retain a 35mm culture dish. This enables a culture dish to be transported with the reader unit to enable monitoring to be continued outside the incubator.

The reader body 21 includes a recess 26 for a cuvette 28 to carry a sample of the fluid which is the same as that in the culture dish as discussed above.

Within the reader body as shown in Figure 3 there is a LED light source arrangement 30 comprised of three or more LEDs of different frequencies as discussed above directed to a light guide 32 so that the light passes across the slot 26 to a LED receiver assembly 36. The LED receiver assembly 36 includes receivers for each of the frequencies of the LED light source arrangement. Electronic circuitry 38 processes the various readings and a battery 39 (underneath the electronic circuitry and shown dotted) makes the reader unit self contained. Adjacent to the light source 30 is a second LED receiver 40 which measures and compensates for drift in the transmitting LED assembly 30. An aerial 42 associated with the electronic circuitry transmits readings to a data logger within the incubator or to a monitoring device outside the incubator. The reader unit also includes a thermocouple 44 to measure temperature and the electronic circuitry 38 can transmit temperature data as well as pH data.

A version of the reader unit as shown in Figures 2 and 3 may be supplied without the gripper. Such a device can be used to monitor a whole incubator chamber and act as a warning device, setting off an alarm when pH or temperature moves outside preset limits.

Throughout this specification various indications have been given as to the scope of this invention but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

## Claims

1. A temperature and pH logging system for cell culture to provide an audit history and/or control feedback for incubation conditions comprising at least one reader unit for measuring pH and temperature associated with the culture media in a culture vessel in an incubator, a data logger for recording and storing the measured pH and temperature to provide the audit history and a warning system if temperature or pH goes outside a specified range, wherein the reader unit comprises a cuvette containing the same culture media as the culture vessel and the measurement of the selected characteristic is carried out in the cuvette.

2. A temperature and pH logging system as in Claim 1 wherein the pH is measured using an optical method of pH measurement comprising using a coloured pH indicator, using three or more wavelengths where two or more are used to derive the pH from a ratio of the acid and base forms of the indicator, so is substantially independent of the amount of indicator present, one or more wavelengths having little absorption by either form of the indicator are used to track and correct for offset changes in zero point conditions, determining calibration coefficients for the zero point as a function of temperature for each of the wavelengths, and applying a zero point correction in conjunction with the temperature measurement, whereby the combination of temperature correction and offset correction of the zero point means it is not necessary to take a zero reading of the solution prior to each measurement.

3. A temperature and pH logging system as in Claim 1 wherein the temperature is measured using a thermocouple.

4. A temperature and pH logging system as in Claim 1 wherein each reader unit is attached to or situated within a particular culture vessel to monitor changes associated with that vessel wherever it is situated.

5. A temperature and pH logging system as in Claim 1 wherein a state of being outside the incubator is determined from an increase in ambient light conditions.

6. A temperature and pH logging system as in Claim 1 wherein the reader unit comprises a reader body, a recess in the reader body to receive the cuvette, a LED arrangement to transmit light through the recess, a LED receiver arrangement to receive light transmitted through the recess, a thermocouple to measure temperature, an electronic circuit to receive and transmit signals from the LED receiver and the thermocouple.

7. A temperature and pH logging system as in Claim 6 wherein the LED arrangement to transmit light comprises at least 3 LEDs providing three or more wavelengths where at least two are used to derive the pH from a ratio of the acid and base forms of a colour indicator and one wavelengths is used to track and correct for offset changes in zero point conditions.

8. A temperature and pH logging system as in Claim 1 wherein the at least one reader unit comprises a gripper arrangement to grip and retain a culture vessel.

9. A temperature and pH logging system as in Claim 1 wherein the at least one reader unit includes wireless transmission means to transmit the measurements to a data logger when the reader unit and culture vessel is inside an incubator and also when the reader unit and culture vessel is outside the incubator.

10. A temperature and pH logging system as in Claim 1 wherein a state of being outside the incubator is determined by a device registering light.

11. A temperature and pH logging system as in Claim 1 further comprising a monitor and display system that includes the warning system to provide an alarm when the pH and temperature are outside the selected range and wherein the warning system provides an alarm that is selected from audible, visual or any other suitable means or combination thereof.

12. A temperature and pH logging system as in Claim 1 wherein the reader unit includes an optical means to measure pH by reading the colour of a pH indicator in media in the cuvette.

## Patentansprüche

1. Temperatur- und pH-Erfassungssystem für eine Zellkultur, das einen Überwachungsverlauf und/oder eine Regelungsrückführung für die Inkubationsbedingungen vorsieht, mit mindestens einer Messsensoreneinheit zur Messung von pH-Wert und Temperatur, die zu dem Kulturmedium in einem Kulturgefäß in einem Inkubator gehören, einem Datensammler zum Aufzeichnen und Speichern der gemessenen pH- und Temperaturwerte für die Bereitstellung des Überwachungsverlaufs und einem Warnsystem, falls die Temperatur oder der pH-Wert aus einem festgelegten Bereich hinauswandert, wobei die Messsensoreneinheit eine Küvette umfasst, welche dasselbe Kulturmedium wie in dem Kulturgefäß enthält und die Messung der ausgewählten Eigenschaft in der Küvette durchgeführt wird.

2. Temperatur- und pH-Erfassungssystem nach Anspruch 1, wobei der pH-Wert unter Verwendung eines optischen Verfahrens der pH-Wert-Messung gemessen wird, umfassend die Verwendung eines farbigen pH-Indikators, die Verwendung von drei oder mehr Wellenlängen, wobei zwei oder mehr Wellenlängen zur Ableitung des pH-Werts aus dem Verhältnis von Säure- und Basenform des Indikators verwendet werden, womit die Messung im Wesentlichen unabhängig von der vorliegenden Indikatormenge ist, wobei eine oder mehrere Wellenlängen, die eine geringe Absorption bei beiden Formen des Indikators aufweisen, zum Verfolgen und Korrigieren der Offset-Verschiebungen von dem Nullpunktzustand, zur Bestimmung der Kalibrierungskoeffizienten für den Nullpunkt als Funktion der Temperatur für jede Wellenlänge und zum Anwenden einer Nullpunktkorrektur in Verbindung mit der Temperaturmessung verwendet werden, wodurch die Kombination aus Temperaturkorrektur und Offset-Korrektur von dem Nullpunkt bedeutet, dass die Durchführung einer Nullablesung von der Lösung vor jeder Messung nicht notwendig ist.

3. Temperatur- und pH-Erfassungssystem nach Anspruch 1, wobei die Temperatur unter Verwendung eines Thermoelements durchgeführt wird.

4. Temperatur- und pH-Erfassungssystem nach Anspruch 1, wobei jede Messsensoreneinheit an einem bestimmten Kulturgefäß zum Überwachen von Änderungen, die mit diesem Kulturgefäß verbunden sind, wo auch immer es aufgestellt ist, befestigt ist oder sich innerhalb von diesem befindet.

5. Temperatur- und pH-Erfassungssystem nach Anspruch 1, wobei ein Zustand, sich außerhalb des Inkubators zu befinden, anhand einer Zunahme der Umgebungslichtverhältnisse bestimmt wird.

6. Temperatur- und pH-Erfassungssystem nach Anspruch 1, wobei die Messsensoreneinheit ein Messsensorengehäuse, eine Aussparung in dem Messsensorengehäuse zur Aufnahme der Küvette, eine LED-Anordnung, um Licht durch die Aussparung zu senden, eine LED-EmpfängerAnordnung zum Empfangen von Licht, das durch die Aussparung gesendet wurde, ein Thermoelement zum Messen der Temperatur und eine elektronische Schaltung zum Empfangen und Senden von Signalen von dem LED-Empfänger und dem Thermoelement umfasst.

7. Temperatur- und pH-Erfassungssystem nach Anspruch 6, wobei die LED-Anordnung zum Senden von Licht mindestens 3 LEDs enthält, die drei oder mehr Wellenlängen bereitstellen, wobei mindestens zwei davon zur Ableitung des pH-Werts aus dem Verhältnis von Säure- und Basenform eines farbigen Indikators verwendet werden und eine Wellenlänge zum Verfolgen und Korrigieren der Offset-Verschiebungen von dem Nullpunktzustand verwendet wird.

8. Temperatur- und pH-Erfassungssystem nach Anspruch 1, wobei die mindestens eine Messsensoreneinheit eine Greifeinrichtung zum Greifen und Festhalten eines Kulturgefäßes umfasst.

9. Temperatur- und pH-Erfassungssystem nach Anspruch 1, wobei die mindestens eine Messsensoreneinheit ein drahtloses Übertragungsmittel zum Senden der Messungen an einen Datensammler umfasst, wenn sich die Messsensoreneinheit und das Kulturgefäß innerhalb eines Inkubators befinden und auch, wenn sich die Messsensoreneinheit und das Kulturgefäß außerhalb des Inkubators befinden.

10. Temperatur- und pH-Erfassungssystem nach Anspruch 1, wobei ein Zustand, sich außerhalb des Inkubators zu befinden, durch eine Vorrichtung zum Erfassen von Licht bestimmt wird.

11. Temperatur- und pH-Erfassungssystem nach Anspruch 1 das ferner einen Monitor und ein Anzeigesystem umfasst, welches das Warnsystem zur Bereitstellung eines Alarms einschließt, wenn der pH-Wert und die Temperatur außerhalb des ausgewählten Bereichs liegen, und wobei das Alarmsystem einen Alarm vorsieht, der ausgewählt ist aus akustischen, visuellen oder irgendwelchen anderen geeigneten Mitteln oder Kombinationen davon.

12. Temperatur- und pH-Erfassungssystem nach Anspruch 1, wobei die Messsensoreneinheit ein optisches Mittel zur Messung des pH-Werts durch Messen der Farbe eines pH-Indikators in den Medien in der Küvette einschließt.

## Revendications

1. Système d'enregistrement de température et de pH pour la culture de cellules destiné à produire un historique d'audit et/ou une information de contrôle des conditions d'incubation, comprenant au moins une unité de lecture pour mesurer le pH et la température associés au milieu de culture dans une cuve de culture à l'intérieur d'un incubateur, un enregistreur de données pour enregistrer et stocker le pH et la température mesurés afin de produire l'historique d' audit et un système d' alerte si la température ou le pH sort d'une plage spécifiée, l'unité de lecture comprenant une cuvette contenant le même milieu de culture que la cuve de culture et la mesure de la caractéristique sélectionnée étant effectuée dans la cuvette.

2. Système d'enregistrement de température et de pH selon la revendication 1, le pH étant mesuré en utilisant un procédé optique de mesure du pH comprenant l'utilisation d'un indicateur de pH coloré, l'utilisation de trois longueurs d'onde ou plus dont deux ou plus sont utilisées pour dériver le pH d'un rapport entre les formes acide et base de l'indicateur de manière à rester sensiblement indépendant de la quantité d'indicateur présente, une ou plusieurs longueurs d'onde ayant une faible absorption par l'une ou l'autre forme de l'indicateur étant utilisées pour suivre et corriger les variations du décalage sous des conditions de point zéro, en déterminant les coefficients de calibrage pour le point zéro en fonction de la température pour chacune des longueurs d'onde et en appliquant une correction de point zéro conjointement avec la mesure de la température, la combinaison de la correction de température et de la correction du décalage du point zéro rendant inutile une lecture du zéro de la solution avant chaque mesure.

3. Système d'enregistrement de température et de pH selon la revendication 1, la température étant mesurée en utilisant un thermocouple.

4. Système d'enregistrement de température et de pH selon la revendication 1, chaque unité de lecture étant fixée ou située à l'intérieur d'une cuve de culture particulière pour surveiller les modifications associées à cette cuve quel que soit l'endroit où elle se trouve.

5. Système d'enregistrement de température et de pH selon la revendication 1, un état à l'extérieur de l'incubateur étant déterminé à partir d'une augmentation des conditions d'éclairage ambiantes.

6. Système d'enregistrement de température et de pH selon la revendication 1, l'unité de lecture comprenant un corps de lecture, un évidement dans le corps de lecture pour accueillir la cuvette, un arrangement à LED pour émettre de la lumière à travers l'évidement, un arrangement de récepteur à LED pour recevoir la lumière émise à travers l'évidement, un thermocouple pour mesurer la température, un circuit électronique pour recevoir et émettre les signaux du récepteur à LED et le thermocouple.

7. Système d'enregistrement de température et de pH selon la revendication 6, l'arrangement à LED pour émettre de la lumière comprenant au moins 3 LED produisant trois longueurs d'onde ou plus dont deux au moins sont utilisées pour dériver le pH d'un rapport entre les formes acide et base d'un indicateur coloré et une longueur d'onde est utilisée pour suivre et corriger les variations du décalage sous des conditions de point zéro.

8. Système d'enregistrement de température et de pH selon la revendication 1, ladite au moins une unité de lecture comprenant un arrangement de pinces pour saisir et retenir une cuve de culture.

9. Système d'enregistrement de température et de pH selon la revendication 1, ladite au moins une unité de lecture comprenant des moyens de transmission sans fil pour transmettre les mesures à un enregistreur de données lorsque l'unité de lecture et la cuve de culture se trouvent à l'intérieur d'un incubateur et aussi lorsque l'unité de lecture et la cuve de culture se trouvent à l'extérieur de l'incubateur.

10. Système d'enregistrement de température et de pH selon la revendication 1, un état à l'extérieur de l'incubateur étant déterminé par un dispositif d'enregistrement de la lumière.

11. Système d'enregistrement de température et de pH selon la revendication 1, comprenant en outre un système de surveillance et d'affichage qui inclut le système d'alerte pour produire une alarme lorsque le pH et la température sont en dehors de la plage choisie et le système d'alerte produisant une alarme qui est choisie parmi des moyens sonores, visuels ou tout autre moyen approprié ou des combinaisons de ceux-ci.

12. Système d'enregistrement de température et de pH selon la revendication 1, l'unité de lecture comprenant des moyens optiques pour mesurer le pH en lisant la couleur d'un indicateur de pH dans le milieu qui se trouve dans la cuvette.
